# EUROPEAN PATENT SPECIFICATION

(11) **EP 3 571 943 B1**
(45) Date of publication and mention of the grant of the patent: **20.01.2021**
(21) Application number: 19179269.6
(22) Date of filing: 20.07.2016
(51) Int. Cl.: A24F 47/00

(54) **ELECTRONIC CIGARETTE**
ELEKTRONISCHE ZIGARETTE
CIGARETTE ÉLECTRONIQUE

(30) Priority: 08.06.2016 CN 201610406045
(43) Date of publication of application: 27.11.2019
(62) Divisional of application: 16901914.8
(73) Proprietor: Joyetech (Changzhou) Electronics Co., Ltd., Changzhou, Jiangsu 213125 (CN)
(72) Inventor: QIU, Weihua, Xinbei District, Changzhou Jiangsu 213125 (CN)
(74) Representative: Haseltine Lake Kempner LLP

(56) References cited:
- WO-A1-2015/120588
- WO-A1-2015/120591
- WO-A1-2015/120636

## Description

### TECHNICAL FIELD

The present application relates to simulated smoking technology, and more particularly to an improved electronic cigarette.

### BACKGROUND OF THE INVENTION

Electronic cigarettes have become a relatively matured substitute for smoking currently on the market. When a heating element of an atomizer of the electronic cigarette is energized by a battery, cigarette liquid is heated by the heating element to generate smoke, such that the user gets the smoking experience.

However, in prior arts, for an electronic cigarette provided with an airflow sensor, the airflow sensor is generally disposed opposite to the mouthpiece. Thus, the airflow sensor is located far away from the mouthpiece, the response speed of the airflow sensor will be influenced, and the user cannot get a favorable smoking experience. Furthermore, the airflow sensor is generally located in the smoke passage, after the airflow sensor is used many times, part of the smoke in the smoke passage will condense into liquid and accumulate on the airflow sensor, which may influence the sensitivity of the airflow sensor.

WO 2015/120588 A1 discloses an electronic cigarette. The electronic cigarette includes an electronic cigarette body. One end of the electronic cigarette body is connected with a sucking nozzle and the other end of the electronic cigarette body is connected with a liquid storage device for containing tobacco liquid. The electronic cigarette body includes an atomization assembly used for atomizing the tobacco liquid and a battery assembly for powering the atomization assembly both arranged between the liquid storage device and the sucking nozzle. The electronic cigarette is provided with a smoke channel and an airflow channel both communicated to the outer surface of the sucking nozzle and spaced apart from each other, and an airflow induction control assembly for controlling the battery assembly to power the atomization assembly is arranged in the airflow channel.

WO 2015/120636 A1 discloses an electronic cigarette having a main body of the electronic cigarette. The main body includes a smoking end, an oil reservoir configured to contain tobacco liquid, an atomizing assembly for atomizing tobacco liquid and a battery assembly for supplying power to the atomizing assembly, all of which are provided on the main body. The oil reservoir is provided on another end of the main body of the electronic cigarette far away from the smoking end.

WO 2015/120591 A1 discloses an electronic cigarette. The electronic cigarette includes an electronic cigarette body on which a smoking end, an oil reservoir used for accommodating tobacco liquid, an atomization assembly used for atomizing the tobacco liquid and a battery pole assembly used for supplying power to the atomization assembly are provided. The oil reservoir is arranged on an end of the electronic cigarette body far away from the smoking end. The electronic cigarette further includes a smoke channel communicated with the smoking end, and an airflow sensor used for generating a smoking signal. The airflow sensor is communicated with the smoke channel through at least two vent holes.

### SUMMARY OF THE INVENTION

In view of the above, it is necessary to provide an electronic cigarette with an improved smoking experience for the user, to satisfy the demand of the market.

The technical proposal of the present application is set forth below:

An electronic cigarette is provided. The invention is defined by the appended claims. The electronic cigarette includes a sensor and an atomizing assembly both of which are disposed in a housing. The atomizing assembly is provided with a first airflow passage. A second airflow passage is provided in the housing, and the sensor is disposed in the second airflow passage. The housing is defined with a first air inlet and a second air inlet. The first air inlet is in fluid communication with the first airflow passage. The second air inlet is in fluid communication with the second airflow passage. The first airflow passage and the second airflow passage are isolated from each other by a partition member. The electronic cigarette further includes a liquid storage unit in fluid communication with the atomizing assembly and an energy device configured for providing power energy for the atomizing assembly. The sensor is triggered to generate a signal to a control board of the energy device when a portion of the external air flows through the sensor.

The atomizing assembly includes an atomizer head and a venting unit. The atomizer head is disposed at a bottom end of the venting unit. The venting unit includes an inner air pipe and an outer air pipe. The inner air pipe is disposed inside the outer air pipe. A gap between the inner air pipe and the outer air pipe forms an air inlet passage. Two ends of the air inlet passage are respectively in fluid communication with the first air inlet and the atomizer head. An air outlet passage is defined inside the inner air pipe. Two ends of the air outlet passage are respectively in fluid communication with the first airflow passage and the atomizer head. The external air enters from the first air inlet, flows downward along the air inlet passage toward the atomizer head, mixes with the smoke generated at the atomizer head, then flows upward along the air outlet passage toward the first airflow passage, and finally exits from the first airflow passage for being inhaled.

The liquid storage unit includes a liquid storage sleeve. The liquid storage sleeve is disposed outside the outer air pipe. A gap between the inner wall of the liquid storage sleeve and the outer wall of the outer air pipe forms a liquid storage chamber for storing cigarette liquid. The liquid storage chamber is in fluid communication with the atomizer head.

Further, the housing includes a main body and a suction body connected at one end of the main body, the partition member is formed in the suction body.

Further, the main body further includes a connection part provided at the end of the main body to which the suction body is connected, the connection part is provided with two mounting holes which are separately formed, the atomizing assembly is mounted in the main body through one of the mounting holes, and the sensor is mounted in the other one of the mounting holes.

Further, the liquid storage unit is disposed in the main body. The liquid storage unit further includes an end sealing element. The end sealing element is mounted at one end of the liquid storage sleeve, and the other end of the liquid storage sleeve is mounted around a bottom side of the mounting hole in which the atomizing assembly is mounted.

Further, the venting unit is provided with a venting hole corresponding to the first air inlet. The venting hole is in fluid communication with the first air inlet and the air inlet passage.

Further, the venting unit further includes a venting connection piece. The venting hole is defined in a sidewall of the venting connection piece. A top end of the inner air pipe and a top end of the outer air pipe are connected with the venting connection piece.

Further, the atomizer head includes an atomizing tube and a heating element. The heating element is disposed in the atomizing tube. A sidewall of the atomizing tube is defined with a liquid inlet hole for the cigarette liquid in the liquid storage chamber to enter into the atomizing tube. The atomizing tube is provided with an atomizing outlet at one end. The atomizing outlet is in fluid communication with the air inlet passage and the air outlet passage.

Further, a top end of the atomizer head is threadedly connected with a bottom end of the outer air pipe.

Further, the atomizer head further includes a liquid absorbing member, the liquid absorbing member wraps up the heating element or the liquid absorbing member is wrapped up by the heating element.

Further, the partition member is integrally formed with the suction body.

Further, the second air inlet is defined in a sidewall or a bottom wall of the housing.

Compared with the prior arts, the electronic cigarette of the present application includes a sensor and an atomizing assembly both of which are disposed in a housing. The atomizing assembly is provided with a first airflow passage. A second airflow passage is provided in the housing, and the sensor is disposed in the second airflow passage. The housing is defined with a first air inlet and a second air inlet. The first air inlet is in fluid communication with the first airflow passage. The second air inlet is in fluid communication with the second airflow passage. The first airflow passage and the second airflow passage are isolated from each other by a partition member. Since the first airflow passage is isolated from the second airflow passage by the partition member, so that the sensor is unaffected when a user inhales smoke from the smoke passage, the sensitivity of the sensor is not reduced, and the smoking experience for the user is improved.

The preferred embodiments of the present application and its advantages are further explained in detail with reference to specific embodiments.

### BRIEF DESCRIPTION OF THE DRAWINGS

The accompanying drawings are intended to provide a further understanding of the present application and constitute a part of the specification, in conjunction with the following specific embodiments for interpretation of the present application, but shall not constitute a limitation to the present application.
FIG. 1 is an isometric view illustrating an electronic cigarette according to an embodiment of the present application;
FIG. 2 is a side view of the electronic cigarette of FIG. 1;
FIG. 3 is a cross sectional view of the electronic cigarette of FIG. 2 along the line of B-B;
FIG. 4 is an exploded view of the electronic cigarette according to the embodiment of the present application;
FIG. 5 is an isometric view of a venting unit according to the embodiment of the present application;
FIG. 6 is a cross sectional view of the electronic cigarette according to the embodiment of the present application;
FIG. 7 is a partially enlarged view of the portion A of FIG. 3;
FIG. 8 is an isometric view of an atomizer head according to the embodiment of the present application;
FIG. 9 is a cross sectional view of the atomizer head of FIG. 8.

In the drawings, the reference numbers are listed below:

battery 1; atomizer head 11; first airflow passage 50; lower air outlet passage 61; control board 2; liquid inlet hole 111; venting hole 51; first air inlet 81; sensor 3; heating element 12; air inlet passage 52; window 82; suction body 4; atomizing base 13; upper air outlet passage 53; end sealing element 21; venting connection piece 5; first contact member 14; upper outer thread 54; partition member 41; inner air pipe 6; second contact member 15; lower outer thread 71; bottom end 181; outer air pipe 7; connecting line 16; atomizing tube 19; liquid storage chamber 91; main body 8; second air inlet 17; venting unit 20; first end 121; liquid storage sleeve 9; connection part 18; lower inner thread 191; second end 122; bottom seat 10; second airflow passage 40; atomizing outlet 192; electronic cigarette 100.

### DETAILED DESCRIPTION OF PREFERRED EMBODIMENTS

The specific embodiments of the present application are described below in detail with reference to the drawings. It should be understood that the specific embodiments described herein are intended only for illustration of the present application, and are not intended to limit the present application.

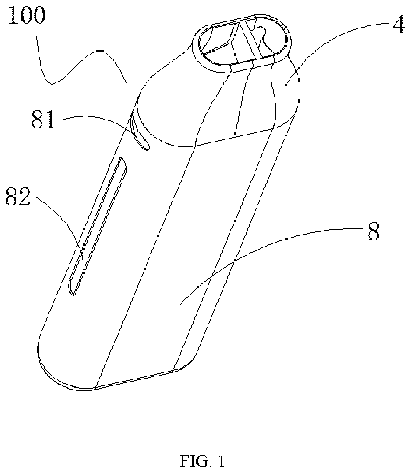

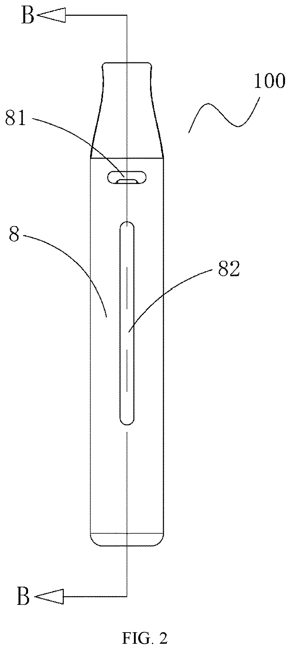

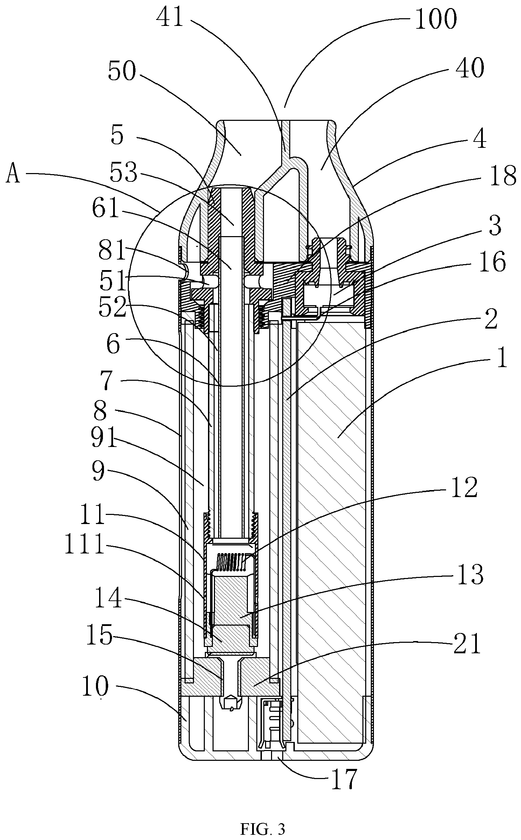

Referring to FIGs. 1-3, an electronic cigarette 100 is provided according to an embodiment of the present application. The electronic cigarette 100 includes an energy device, a sensor 3, an atomizing assembly, and a liquid storage unit. The energy device, the sensor 3, the atomizing assembly and the liquid storage unit are disposed in a housing. The sensor 3 is signal connected with the energy device. Specifically, referring to FIG. 3, the energy device includes a battery 1 and a control board 2. The sensor 3 may connect with the control board 2 by a connecting line 16, and the control board 2 is electrically connected to the battery 1. When the sensor 3 sends out control signals to the control board 2, the control board 2 controls the battery 1 to energize the atomizing assembly, and the electronic cigarette 100 performs the atomizing function.

According to an example of the embodiment, the energy device can also be an apparatus that adopts chemical reaction or infrared radiation to generate heat.

Referring to FIGs. 3-6, according to the electronic cigarette 100 illustrated in the embodiment of the present application, the atomizing assembly is provided with a first airflow passage 50, a second airflow passage 40 is provided in the housing, and the sensor 3 is disposed in the second airflow passage 40. The housing is defined with a first air inlet 81 and a second air inlet 17. The first air inlet 81 is in fluid communication with the first airflow passage 50. The second air inlet 17 is in fluid communication with the second airflow passage 40. The first airflow passage 50 and the second airflow passage 40 are isolated from each other by a partition member 41.

It can be seen from the above, in the electronic cigarette 100 provided by the embodiment, the first airflow passage 50 is isolated from the second airflow passage 40 by the partition member 41. Thus, in the use of the electronic cigarette 100, the smoke generated by the atomizing assembly flows in the first airflow passage 50. However, the second airflow passage 40, in which the sensor 3 is disposed, is isolated from the first airflow passage 50 by the partition member 41, such that the airflows in the two airflow passages are not mixed before being inhaled by the user. That is, the smoke will not flow through the second airflow passage 40. Therefore, even if the smoke condenses, its influence on the sensitivity of the sensor 3 is greatly reduced. Accordingly, the sensor 3 can sensitively control the battery 1 to energize or not. As a result, the smoking experience for the user is improved.

According to an example of the embodiment, referring to FIGs. 3-5, the aforementioned housing includes a main body 8 and a suction body 4 connected at one end of the main body 8. The partition member 41 is formed in the suction body 4. Through forming the housing by separate parts, the producing and assembling of the housing becomes relatively simple, and the quality of the product is improved. The atomizing assembly, the sensor and other parts are assembled in the main body 8, and finally the suction body 4 is mounted, it is therefore convenient to check the states of all of the parts. Further, when the suction body 4 needs to be replaced, it is also convenient. Preferably, the partition member 41 is integrally formed with the suction body 4, to thereby reduce the manufacturing steps.

According to an example of the embodiment, the main body 8 further includes a connection part 18 provided at the end of the main body 8 to which the suction body 4 is connected. In the connection part 18, there are provided with two mounting holes 182, 183 which are separately formed, wherein the atomizing assembly is mounted in the main body 8 through one of the mounting holes 182, 183, and the sensor 3 is mounted in the other one of the mounting holes 182, 183. Thus, through forming two mounting holes 182, 183 in the connection part 18, the atomizing assembly and the sensor 3 are further isolated, to ensure the first airflow passage 50 and the second airflow passage 40 are not overlapped.

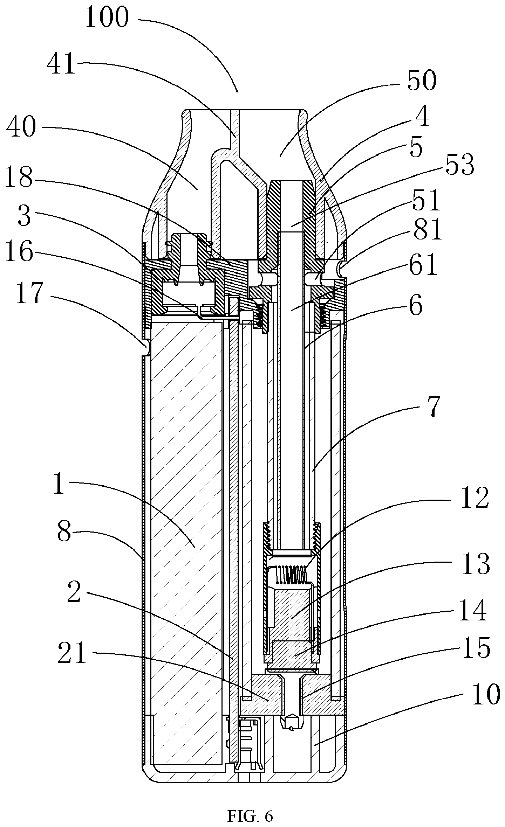

Referring to FIG. 3 and FIG. 6, according to an example of the embodiment, the main body 8 further includes a bottom seat 10. The bottom seat 10 is provided at the bottom end of the main body 8. The second air inlet 17 can be defined in the bottom seat 10. Alternatively, as shown in FIG. 6, the second air inlet 17 can also be defined in a sidewall of the main body 8. When the second air inlet 17 is defined in the sidewall of the main body 8, the second air inlet 17 is located closer to the suction body 4 of the electronic cigarette 100. Thus, the resistance is relatively low, and the sensor 3 can have a higher sensitivity.

According to an example of the embodiment, referring to FIG. 3, the atomizing assembly includes an atomizer head 11 and a venting unit 20. The atomizer head 11 is disposed at one end of the venting unit 20. The venting unit 20 is provided with a lower air outlet passage 61 in fluid communication with the first airflow passage 50. The venting unit 20 is further provided with a venting hole 51 in fluid communication with the first air inlet 81. According to an example, the venting unit 20 includes a venting connection piece 5, an inner air pipe 6, and an outer air pipe 7, wherein the inner air pipe 6 and the outer air pipe 7 are connected with the venting connection piece 5. The inner air pipe 6 is disposed inside the outer air pipe 7. The gap between the inner air pipe 6 and the outer air pipe 7 forms as an air inlet passage 52. The air inlet passage 52 is in fluid communication with the venting hole 51. The lower air outlet passage 61 is formed by an interior passage of the inner air pipe 6. In the venting connection piece 5, there is provided with an upper air outlet passage 53. The air inlet passage 52, the lower air outlet passage 61 and the upper air outlet passage 53 are in fluid communication with the first airflow passage 50.

Referring to FIGs. 3-9, according to an example of the embodiment, when the user performs inhaling operation on the suction body 4 of the electronic cigarette 100, due to the existence of the partition member 41, the external air is inhaled by the user from two separate paths. In one of the paths, a portion of the external air enters from the first air inlet 81, and then flows through the venting hole 51, the air inlet passage 52, the lower air outlet passage 61 and the upper air outlet passage 53 to reach the first airflow passage 50. In the other one of the paths, a portion of the external air enters into the housing from the second air inlet 17, and then flows through the sensor 3 to reach the second airflow passage 40. As the portion of the external air flows through the sensor 3, the sensor 3 is triggered to generate a signal to the control board 2 of the energy device, to control the battery 1 to output energy power. It is noted that, the external air flows along the two paths concurrently and separately, wherein the external air flowing through the sensor 3 can trigger the sensor 3 to generate a signal to the control board 2 of the energy device, so that the battery 1 is controlled to output energy power. After the atomizing assembly gets the energy power from the battery 1, the atomizer head 11 of the atomizing assembly uses the heating element 12 to heat and atomize the cigarette liquid to generate smoke. The generated smoke discharges out from an atomizing outlet 192, and the external air after entering from the first air inlet 81 and flowing through the air inlet passage 52 can take away the generated smoke. The external air and the smoke are mixed together and flow through the lower air outlet passage 61 and the upper air outlet passage 53 to reach the first airflow passage 50, for finally being inhaled by the user.

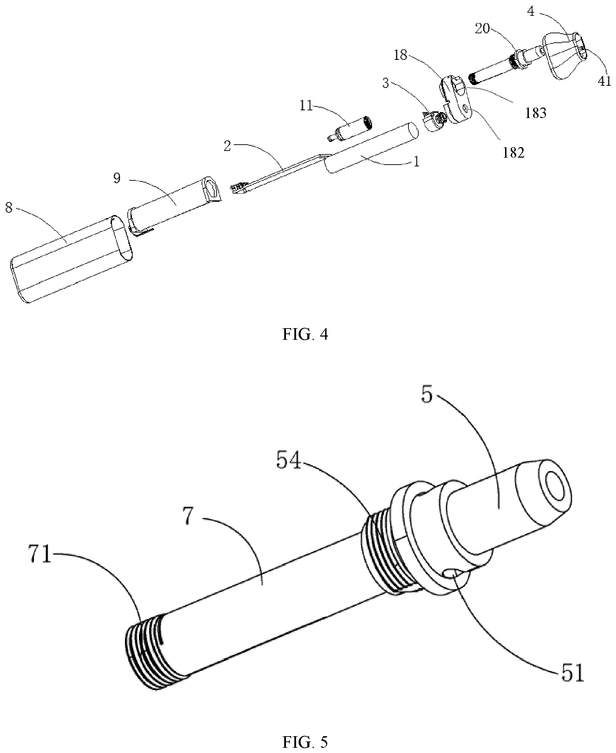

Referring to FIGs. 5-9, according to an example of the embodiment, the atomizer head 11 includes an atomizing tube 19, a heating element 12, an atomizing base 13, and a first contact member 14. The heating element 12, the atomizing base 13 and the first contact member 14 are disposed in the atomizing tube 19. The heating element 12 is located above the atomizing base 13. The atomizing tube 19 is provided with the atomizing outlet 192 at one end thereof. The atomizing base 13 is disposed at the other end of the atomizing tube 19 opposite to the atomizing outlet 192. The first contact member 14 is disposed at a bottom end of the atomizing base 13.

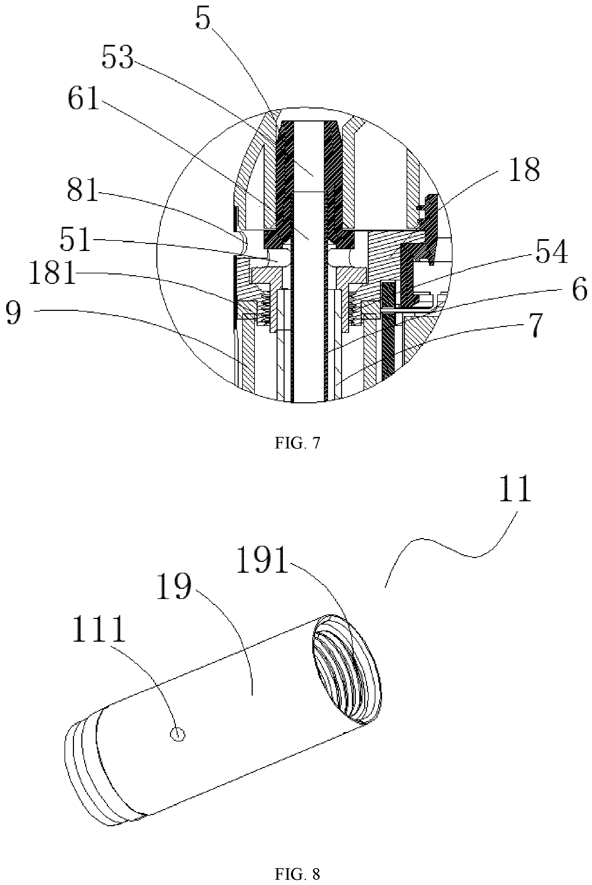

According to an example of the embodiment, a top end of the atomizer head 11 is threadedly connected with a bottom end of the outer air pipe 7. Particularly, referring to FIG. 5 and FIG. 8, the top end of the atomizer head 11 is provided with a lower inner thread 191, and the bottom end of the outer air pipe 7 of the venting unit 20 is provided with a lower outer thread 71 engageable with the lower inner thread 191, such that the atomizer head 11 can be fixed to the bottom end of the venting unit 20. The atomizing outlet 192 is in fluid communication with the air inlet passage 52 and the lower air outlet passage 61. In the use of the electronic cigarette, a portion of the external air enters into the atomizing assembly from the first air inlet 81, the venting hole 51 and the air inlet passage 52. The atomizer head 11 heats and atomizes the cigarette liquid to generate smoke, the generated smoke is taken away by this portion of the external air and then flows through the lower air outlet passage 61 and the upper air outlet passage 53 to enter into the first airflow passage 50, finally for the user to inhale. The atomizer head 11 further includes a liquid absorbing member, wherein the liquid absorbing member wraps up the heating element 12, or the liquid absorbing member is wrapped up by the heating element 12. The material for the liquid absorbing member can be porous ceramics, cotton, cotton cloth, porous metals, etc., so long as the liquid absorbing member can absorb the cigarette liquid and store a certain amount of the cigarette liquid.

Referring to FIG. 3 and FIG. 7, the venting hole 51 is defined in a sidewall of the venting connection piece 5. The inner air pipe 6 and the outer air pipe 7 are disposed at a lower end of the venting connection piece 5. The liquid storage unit is disposed in the main body 8. The liquid storage unit includes a liquid storage sleeve 9 and an end sealing element 21. The gap between the inner wall of the liquid storage sleeve 9 and the outer wall of the outer air pipe 7 forms as a liquid storage chamber 91. The end sealing element 21 is provided at one end of the liquid storage sleeve 9, and the other end of the liquid storage sleeve 9 is mounted around a bottom side of the mounting hole 183 in which the atomizing assembly is mounted.

Referring to FIG. 5 and FIG. 7, the bottom end of the venting connection piece 5 is provided with an upper outer thread 54. The mounting hole 183, in which the atomizing assembly is mounted, is provided with an upper inner thread engaged with the upper outer thread 54, as shown in FIG. 7. In addition, according to an example of the embodiment, the top end of the liquid storage sleeve 9 is connected to a bottom end 181 of the connection part 18 by an interference fit, a silicon gasket can be mounted therebetween for preventing the cigarette liquid from leakage. A sidewall of the atomizing tube 19 is defined with a liquid inlet hole 111, such that the cigarette liquid in the liquid storage chamber 91 can enter from the liquid inlet hole 111 into an interior of the atomizer head 11 to be heated and atomized by the heating element 12.

According to an example of the embodiment, referring to FIGs. 1-3, the sidewall of the main body 8 is provided with a window 82. The liquid storage sleeve 9 may be made of a transparent or translucent material. From the window 82, the cigarette liquid in the liquid storage chamber 91 can be observed through the liquid storage sleeve 9 made of a transparent or translucent material, to prevent a dry burning problem occurred when the liquid storage chamber 91 of the electronic cigarette has no cigarette liquid or is lack of cigarette liquid.

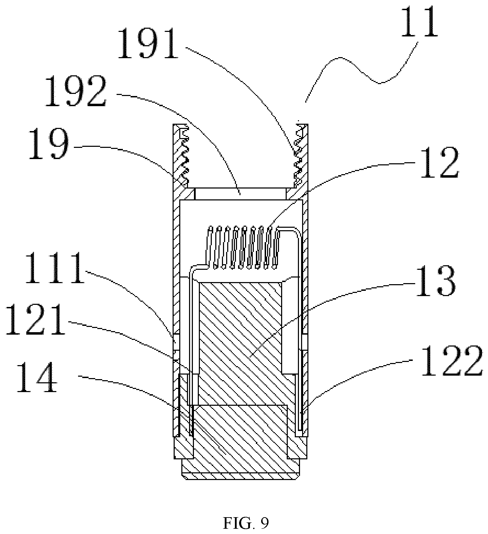

Referring to FIG. 3 and FIG. 9, the liquid storage unit further includes a second contact member 15 mounted on the end sealing element 21. The second contact member 15 is electrically connected to the first contact member 14. The first contact member 14 can be electrically connected to an electrode of the battery 1. Thus, a first end 121 of the heating element 12 in the atomizer head 11 can be electrically connected to an electrode of the battery 1 by the first contact member 14 and the second contact member 15, and a second end 122 of the heating element 12 can be electrically connected to the other electrode of the battery 1 through other ways. For example, the atomizing tube 19, the outer air pipe 7, the venting connection piece 5, the connection part 18 and other related parts can be made of electrically conductive materials, and the second end 122 of the heating element 12 is electrically connected to the other electrode of the battery 1 after electrically connecting with the atomizing tube 19. It is well known by a person skilled in the art, there are other methods to realize the electrical connection, for example, a relatively simple method is to use an electrical wire to connect the second end 122 of the heating element 12 with the other electrode of the battery 1.

The above embodiments described in detail are several embodiments of the present application only, and should not be deemed as limitations to the scope of the present application. Therefore, the scope of the present application is defined by the appended claims.

## Claims

1. An electronic cigarette (100), comprising a sensor (3) and an atomizing assembly both of which are disposed in a housing, wherein the atomizing assembly is provided with a first airflow passage (50), a second airflow passage (40) is provided in the housing, and the sensor (3) is disposed in the second airflow passage (40), the housing is defined with a first air inlet (81) and a second air inlet (17), the first air inlet (81) is in fluid communication with the first airflow passage (50), the second air inlet (17) is in fluid communication with the second airflow passage (40), the first airflow passage (50) and the second airflow passage (40) are isolated from each other by a partition member (41), the electronic cigarette (100) further comprises a liquid storage unit in fluid communication with the atomizing assembly and an energy device configured for providing power energy for the atomizing assembly, the sensor (3) is triggered to generate a signal to a control board (2) of the energy device when a portion of the external air flows through the sensor (3);
**characterized in that** the atomizing assembly comprises an atomizer head (11) and a venting unit (20), the atomizer head (11) is disposed at a bottom end of the venting unit (20), the venting unit (20) comprises an inner air pipe (6) and an outer air pipe (7), the inner air pipe (6) is disposed inside the outer air pipe (7), a gap between the inner air pipe (6) and the outer air pipe (7) forms an air inlet passage (52), two ends of the air inlet passage (52) are respectively in fluid communication with the first air inlet (81) and the atomizer head (11), an air outlet passage is defined inside the inner air pipe (6), two ends of the air outlet passage are respectively in fluid communication with the first airflow passage (50) and the atomizer head (11), whereby the external air enters from the first air inlet (81), flows downward along the air inlet passage (52) toward the atomizer head (11), mixes with the smoke generated at the atomizer head (11), then flows upward along the air outlet passage toward the first airflow passage (50), and finally exits from the first airflow passage (50) for being inhaled;
the liquid storage unit comprises a liquid storage sleeve (9), the liquid storage sleeve (9) is disposed outside the outer air pipe (7), a gap between the inner wall of the liquid storage sleeve (9) and the outer wall of the outer air pipe (7) forms a liquid storage chamber (91) for storing cigarette liquid, the liquid storage chamber (91) is in fluid communication with the atomizer head (11).

2. The electronic cigarette (100) of claim **1,** wherein the housing comprises a main body (8) and a suction body (4) connected at one end of the main body (8), and the partition member (41) is formed in the suction body (4).

3. The electronic cigarette (100) of claim **2,** wherein the main body (8) comprises a connection part (18) provided at the end of the main body (8) to which the suction body (4) is connected, the connection part (18) is provided with two mounting holes (182, 183) which are separately formed, the atomizing assembly is mounted in the main body (8) through one of the mounting holes (182, 183), the sensor (3) is mounted in the other one of the mounting holes (182, 183).

4. The electronic cigarette (100) of claim **3,** wherein the liquid storage unit is disposed in the main body (8), the liquid storage unit further comprises an end sealing element (21), the end sealing element (21) is mounted at one end of the liquid storage sleeve (9), and the other end of the liquid storage sleeve (9) is mounted around a bottom side of the mounting hole (183) in which the atomizing assembly is mounted.

5. The electronic cigarette (100) of claim **1,** wherein the venting unit (20) is provided with a venting hole (51) corresponding to the first air inlet (81), the venting hole (51) is in fluid communication with the first air inlet (81) and the air inlet passage (52).

6. The electronic cigarette (100) of claim **5,** wherein the venting unit (20) further comprises a venting connection piece (5), the venting hole (51) is defined in a sidewall of the venting connection piece (5), a top end of the inner air pipe (6) and a top end of the outer air pipe (7) are connected with the venting connection piece (5).

7. The electronic cigarette (100) of claim **1,** wherein the atomizer head (11) comprises an atomizing tube (19) and a heating element (12), the heating element (12) is disposed in the atomizing tube (19), a sidewall of the atomizing tube (19) is defined with a liquid inlet hole (111) for the cigarette liquid in the liquid storage chamber (91) to enter into the atomizing tube (19), the atomizing tube (19) is provided with an atomizing outlet (192) at one end, the atomizing outlet (192) is in fluid communication with the air inlet passage (52) and the air outlet passage.

8. The electronic cigarette (100) of claim **7,** wherein a top end of the atomizing tube (19) is threadedly connected with a bottom end of the outer air pipe (7).

9. The electronic cigarette (100) of claim **7,** wherein the atomizing head (11) further comprises a liquid absorbing member, the liquid absorbing member wraps up the heating element (12) or the liquid absorbing member is wrapped up by the heating element (12).

10. The electronic cigarette (100) of claim **2,** wherein the partition member (41) is integrally formed with the suction body (4).

11. The electronic cigarette (100) of claim **1,** wherein the second air inlet (17) is defined in a sidewall or a bottom wall of the housing.

## Patentansprüche

1. Elektronische Zigarette (100), umfassend einen Sensor (3) und eine Zerstäubungsanordnung, die beide in einem Gehäuse angeordnet sind, wobei die Zerstäubungsanordnung mit einem ersten Luftstromdurchgang (50) versehen ist, ein zweiter Luftstromdurchgang (40) in dem Gehäuse bereitgestellt ist und der Sensor (3) in dem zweiten Luftstromdurchgang (40) angeordnet ist, das Gehäuse mit einem ersten Lufteinlass (81) und einem zweiten Lufteinlass (17) definiert ist, der erste Lufteinlass (81) in Fluidverbindung mit dem ersten Luftstromdurchgang (50) ist, der zweite Lufteinlass (17) in Fluidverbindung mit dem zweiten Luftstromdurchgang (40) ist, der erste Luftstromdurchgang (50) und der zweite Luftstromdurchgang (40) durch ein Trennelement (41) voneinander isoliert sind, wobei die elektronische Zigarette (100) ferner eine Flüssigkeitsspeichereinheit in Fluidverbindung mit der Zerstäubungsanordnung und eine Energievorrichtung, die konfiguriert ist, um Leistungsenergie für die Zerstäubungsanordnung bereitzustellen, umfasst, wobei der Sensor (3) ausgelöst wird, um ein Signal an eine Steuerplatine (2) der Energievorrichtung zu erzeugen, wenn ein Teil der Außenluft durch den Sensor (3) strömt;
**dadurch gekennzeichnet, dass** die Zerstäubungsanordnung einen Zerstäuberkopf (11) und eine Entlüftungseinheit (20) umfasst, der Zerstäuberkopf (11) an einem unteren Ende der Entlüftungseinheit (20) angeordnet ist, die Entlüftungseinheit (20) ein inneres Luftrohr (6) und ein äußeres Luftrohr (7) umfasst, das innere Luftrohr (6) innerhalb des äußeren Luftrohrs (7) angeordnet ist, ein Spalt zwischen dem inneren Luftrohr (6) und dem äußeren Luftrohr (7) einen Lufteinlassdurchgang (52) bildet, zwei Enden des Lufteinlassdurchgangs (52) jeweils in Fluidverbindung mit dem ersten Lufteinlass (81) und dem Zerstäuberkopf (11) sind, ein Luftauslassdurchgang innerhalb des inneren Luftrohrs (6) definiert ist, zwei Enden des Luftauslassdurchgangs jeweils in Fluidverbindung mit dem ersten Luftstromdurchgang (50) und dem Zerstäuberkopf (11) sind, wodurch die Außenluft von dem ersten Lufteinlass (81) eintritt, entlang des Lufteinlassdurchgangs (52) nach unten zu dem Zerstäuberkopf (11) strömt, sich mit dem an dem Zerstäuberkopf (11) erzeugten Rauch vermischt, dann entlang des Luftauslassdurchgangs nach oben zu dem ersten Luftstromdurchgang (50) strömt, und schließlich aus dem ersten Luftstromdurchgang (50) austritt, um inhaliert zu werden;
die Flüssigkeitsspeichereinheit eine Flüssigkeitsspeicherhülse (9) umfasst, die Flüssigkeitsspeicherhülse (9) außerhalb des äußeren Luftrohrs (7) angeordnet ist, ein Spalt zwischen der Innenwand der Flüssigkeitsspeicherhülse (9) und der Außenwand des äußeren Luftrohrs (7) eine Flüssigkeitsspeicherkammer (91) zum Speichern von Zigarettenflüssigkeit bildet, die Flüssigkeitsspeicherkammer (91) in Fluidverbindung mit dem Zerstäuberkopf (11) ist.

2. Elektronische Zigarette (100) gemäß Anspruch 1, wobei das Gehäuse einen Hauptkörper (8) und einen Saugkörper (4), der mit einem Ende des Hauptkörpers (8) verbunden ist, aufweist, und das Trennelement (41) in dem Saugkörper (4) gebildet ist.

3. Elektronische Zigarette (100) gemäß Anspruch 2, wobei der Hauptkörper (8) ein Verbindungsteil (18) umfasst, das an dem Ende des Hauptkörpers (8) bereitgestellt ist, mit dem der Saugkörper (4) verbunden ist, das Verbindungsteil (18) mit zwei Montagelöchern (182, 183) versehen ist, die separat gebildet sind, die Zerstäubungsanordnung durch eines der Montagelöcher (182, 183) in dem Hauptkörper (8) montiert ist, der Sensor (3) in dem anderen der Montagelöcher (182, 183) montiert ist.

4. Elektronische Zigarette (100) gemäß Anspruch 3, wobei die Flüssigkeitsspeichereinheit in dem Hauptkörper (8) angeordnet ist, die Flüssigkeitsspeichereinheit ferner ein Endabdichtungselement (21) umfasst, das Endabdichtungselement (21) an einem Ende der Flüssigkeitsspeicherhülse (9) montiert ist und das andere Ende der Flüssigkeitsspeicherhülse (9) um eine Unterseite des Montagelochs (183) montiert ist, in dem die Zerstäubungsanordnung montiert ist.

5. Elektronische Zigarette (100) gemäß Anspruch 1, wobei die Entlüftungseinheit (20) mit einem Entlüftungsloch (51) versehen ist, das dem ersten Lufteinlass (81) entspricht, das Entlüftungsloch (51) in Fluidverbindung mit dem ersten Lufteinlass (81) und dem Lufteinlassdurchgang (52) ist.

6. Elektronische Zigarette (100) gemäß Anspruch 5, wobei die Entlüftungseinheit (20) ferner ein Entlüftungsverbindungsstück (5) umfasst, das Entlüftungsloch (51) in einer Seitenwand des Entlüftungsverbindungsstücks (5) definiert ist, ein oberes Ende des inneren Luftrohrs (6) und ein oberes Ende des äußeren Luftrohrs (7) mit dem Entlüftungsverbindungsstück (5) verbunden sind.

7. Elektronische Zigarette (100) gemäß Anspruch 1, wobei der Zerstäuberkopf (11) ein Zerstäubungsrohr (19) und ein Heizelement (12) umfasst, das Heizelement (12) in dem Zerstäubungsrohr (19) angeordnet ist, eine Seitenwand des Zerstäubungsrohrs (19) mit einem Flüssigkeitseinlassloch (111) für die Zigarettenflüssigkeit in der Flüssigkeitsspeicherkammer (91) definiert ist, um in das Zerstäubungsrohr (19) einzutreten, das Zerstäubungsrohr (19) mit einem Zerstäubungsauslass (192) an einem Ende versehen ist, der Zerstäubungsauslass (192) in Fluidverbindung mit dem Lufteinlassdurchgang (52) und dem Luftauslassdurchgang ist.

8. Elektronische Zigarette (100) gemäß Anspruch 7, wobei ein oberes Ende des Zerstäubungsrohrs (19) mit einem unteren Ende des äußeren Luftrohrs (7) verschraubt ist.

9. Elektronische Zigarette (100) gemäß Anspruch 7, wobei der Zerstäuberkopf (11) ferner ein flüssigkeitsabsorbierendes Element umfasst, das flüssigkeitsabsorbierende Element das Heizelement (12) umhüllt oder das flüssigkeitsabsorbierende Element von dem Heizelement (12) umhüllt ist.

10. Elektronische Zigarette (100) gemäß Anspruch 2, wobei das Trennelement (41) einteilig mit dem Saugkörper (4) gebildet ist.

11. Elektronische Zigarette (100) gemäß Anspruch 1, wobei der zweite Lufteinlass (17) in einer Seitenwand oder einer Bodenwand des Gehäuses definiert ist.

## Revendications

1. Une cigarette électronique (100), comprenant un capteur (3) et un ensemble d'atomisation qui sont tous deux disposés dans un boîtier, dans laquelle l'ensemble d'atomisation est prévu avec un premier passage d'écoulement d'air (50), un deuxième passage d'écoulement d'air (40) est prévu dans le boîtier, et le capteur (3) est disposé dans le deuxième passage d'écoulement d'air (40), le boîtier est défini avec une première entrée d'air (81) et une deuxième entrée d'air (17), la première entrée d'air (81) est en communication fluidique avec le premier passage d'écoulement d'air (50), la deuxième entrée d'air (17) est en communication fluidique avec le deuxième passage d'écoulement d'air (40), le premier passage d'écoulement d'air (50) et le deuxième passage d'écoulement d'air (40) sont isolés l'un de l'autre par un élément de séparation (41), la cigarette électronique (100) comprend en outre une unité de stockage de liquide en communication fluidique avec l'ensemble d'atomisation et un dispositif d'énergie configuré pour fournir de l'énergie électrique pour l'ensemble d'atomisation, le capteur (3) est déclenché pour générer un signal vers un panneau de contrôle (2) du dispositif d'énergie lorsqu'une partie de l'air externe s'écoule à travers le capteur (3) ;
**caractérisé en ce que** l'ensemble d'atomisation comprend une tête d'atomiseur (11) et une unité de ventilation (20), la tête d'atomiseur (11) est disposée à une extrémité inférieure de l'unité de ventilation (20), l'unité de ventilation (20) comprend un tuyau d'air intérieur (6) et un tuyau d'air extérieur (7), le tuyau d'air intérieur (6) est disposé à l'intérieur du tuyau d'air extérieur (7), un espace entre le tuyau d'air intérieur (6) et le tuyau d'air extérieur (7) forme un passage d'entrée d'air (52), deux extrémités du passage d'entrée d'air (52) sont respectivement en communication fluidique avec la première entrée d'air (81) et la tête d'atomiseur (11), un passage de sortie d'air est défini à l'intérieur du tuyau d'air intérieur (6), deux extrémités du passage de sortie d'air sont respectivement en communication fluidique avec le premier passage d'écoulement d'air (50) et la tête d'atomiseur (11), par lesquelles l'air externe entre à partir de la première entrée d'air (81), s'écoule vers le bas le long du passage d'entrée d'air (52) vers la tête d'atomiseur (11), se mélange avec la fumée générée à la tête d'atomiseur (11), puis s'écoule vers le haut le long du passage de sortie d'air vers le premier passage d'écoulement d'air (50), et enfin sort à partir du premier passage d'écoulement d'air (50) pour être inhalé ;
l'unité de stockage de liquide comprend un manchon de stockage de liquide (9), le manchon de stockage de liquide (9) est disposé à l'extérieur du tuyau d'air extérieur (7), un espace entre la paroi intérieure du manchon de stockage de liquide (9) et la paroi extérieure du tuyau d'air extérieur (7) forme une chambre de stockage de liquide (91) pour stocker le liquide de cigarette, la chambre de stockage de liquide (91) est en communication fluidique avec la tête d'atomiseur (11).

2. La cigarette électronique (100) selon la revendication 1, dans laquelle le boîtier comprend un corps principal (8) et un corps d'aspiration (4) connecté à une extrémité du corps principal (8), et l'élément de séparation (41) est formé dans le corps d'aspiration (4).

3. La cigarette électronique (100) selon la revendication 2, dans laquelle le corps principal (8) comprend une partie de connexion (18) prévue à l'extrémité du corps principal (8) à laquelle est connecté le corps d'aspiration (4), la partie de connexion (18) est prévue avec deux trous de montage (182, 183) qui sont formés séparément, l'ensemble d'atomisation est monté dans le corps principal (8) à travers un des trous de montage (182, 183), le capteur (3) est monté dans l'autre des trous de montage (182, 183).

4. La cigarette électronique (100) selon la revendication 3, dans laquelle l'unité de stockage de liquide est disposée dans le corps principal (8), l'unité de stockage de liquide comprend en outre un élément d'étanchéité d'extrémité (21), l'élément d'étanchéité d'extrémité (21) est monté à une extrémité du manchon de stockage de liquide (9), et l'autre extrémité du manchon de stockage de liquide (9) est montée autour d'un côté inférieur du trou de montage (183) dans lequel l'ensemble d'atomisation est monté.

5. La cigarette électronique (100) selon la revendication 1, dans laquelle l'unité de ventilation (20) est prévue avec un trou de ventilation (51) correspondant à la première entrée d'air (81), le trou de ventilation (51) est en communication fluidique avec la première entrée d'air (81) et le passage d'entrée d'air (52).

6. La cigarette électronique (100) selon la revendication 5, dans laquelle l'unité de ventilation (20) comprend en outre une pièce de connexion de ventilation (5), le trou de ventilation (51) est défini dans une paroi latérale de la pièce de connexion de ventilation (5), une extrémité supérieure du tuyau d'air intérieur (6) et une extrémité supérieure du tuyau d'air extérieur (7) sont connectées avec la pièce de connexion de ventilation (5).

7. La cigarette électronique (100) selon la revendication 1, dans laquelle la tête d'atomiseur (11) comprend un tube d'atomisation (19) et un élément de chauffage (12), l'élément de chauffage (12) est disposé dans le tube d'atomisation (19), une paroi latérale du tube d'atomisation (19) est définie avec un trou d'entrée de liquide (111) pour que le liquide de cigarette dans la chambre de stockage de liquide (91) entre dans le tube d'atomisation (19), le tube d'atomisation (19) est prévu avec une sortie d'atomisation (192) à une extrémité, la sortie d'atomisation (192) est en communication fluidique avec le passage d'entrée d'air (52) et le passage de sortie d'air.

8. La cigarette électronique (100) selon la revendication 7, dans laquelle une extrémité supérieure du tube d'atomisation (19) est connectée par filetage à une extrémité inférieure du tuyau d'air extérieur (7).

9. La cigarette électronique (100) selon la revendication 7, dans laquelle la tête d'atomisation (11) comprend en outre un élément d'absorption de liquide, l'élément d'absorption de liquide enveloppe l'élément de chauffage (12) ou l'élément d'absorption de liquide est enveloppé par l'élément de chauffage (12).

10. La cigarette électronique (100) selon la revendication 2, dans laquelle l'élément de séparation (41) est formé intégralement avec le corps d'aspiration (4).

11. La cigarette électronique (100) selon la revendication 1, dans laquelle la deuxième entrée d'air (17) est définie dans une paroi latérale ou une paroi inférieure du boîtier.
